# EUROPEAN PATENT SPECIFICATION

(11) **EP 1 750 633 B1**
(45) Date of publication and mention of the grant of the patent: **25.02.2015**
(21) Application number: 05754370.4
(22) Date of filing: 17.05.2005
(51) Int. Cl.: A61F 9/008, A61B 3/00

(54) **BINOCULAR OPTICAL TREATMENT FOR PRESBYOPIA**
BINOKULARE OPTISCHE BEHANDLUNG VON PRESBYOPIE
TRAITEMENT OPTIQUE BINOCULAIRE POUR LA PRESBYTIE

(30) Priority: 18.05.2004 US 849573
(43) Date of publication of application: 14.02.2007
(73) Proprietor: AMO Manufacturing USA, LLC, Santa Ana, CA 92705-4933 (US)
(72) Inventor: CHERNYAK, Dimitri, Santa Clara, California 95051 (US)
(74) Representative: Kazi, Ilya
(86) International application number: PCT/US2005/017991
(87) International publication number: WO 2005/112844

(56) References cited:
- WO-A1-99/44492
- WO-A1-2004/052253
- WO-A2-03/077795
- US-A- 4 732 148
- US-A1- 2002 156 467
- US-A1- 2004 054 356
- US-B1- 6 312 424

## Description

### BACKGROUND OF THE INVENTION

This invention generally relates to methods and systems for providing optical correction. More particularly, the invention provides methods and systems for mitigating or treating presbyopia and other vision conditions.

Presbyopia is a condition that affects the accommodation properties of the eye. As objects move closer to a young, properly functioning eye, the effects of ciliary muscle contraction and zonular relaxation allow the lens of the eye to become rounder or more convex, and thus increase its optical power and ability to focus at near distances. Accommodation can allow the eye to focus and refocus between near and far objects.

Presbyopia normally develops as a person ages, and is associated with a natural progressive loss of accommodation, sometimes referred to as "old sight." The presbyopic eye often loses the ability to rapidly and easily refocus on objects at varying distances. There may also be a loss in the ability to focus on objects at near distances. Although the condition progresses over the lifetime of an individual, the effects of presbyopia usually become noticeable after the age of 45 years. By the age of 65 years, the crystalline lens has often lost almost all elastic properties and has only limited ability to change shape. Residual accommodation refers to the amount of accommodation that remains in the eye. A lower degree of residual accommodation contributes to more severe presbyopia, whereas a higher amount of residual accommodation correlates with less severe presbyopia.

Known methods and devices for treating presbyopia seek to provide vision approaching that of an emmetropic eye. In an emmetropic eye, both distant objects and near objects can be seen due to the accommodation properties of the eye. To address the vision problems associated with presbyopia, reading glasses have traditionally been used by individuals to add plus power diopter to the eye, thus allowing the eye to focus on near objects and maintain a clear image. This approach is similar to that of treating hyperopia, or farsightedness.

Presbyopia has also been treated with bi-focal eyeglasses, where one portion of the lens is corrected for distance vision, and another portion of the lens is corrected for near vision. When peering down through the bifocals, the individual looks through the portion of the lens corrected for near vision. When viewing distant objects, the individual looks higher, through the portion of the bi-focals corrected for distance vision. Thus with little or no accommodation, the individual can see both far and near objects.

Contact lenses and intra-ocular lenses (IOLs) have also been used to treat presbyopia. One approach is to provide the individual with monovision, where one eye (usually the primary eye) is corrected for distance-vision, while the other eye is corrected for near-vision. Unfortunately, with monovision the individual may not clearly see objects that are intermediately positioned because the object is out-of-focus for both eyes. Also, an individual may have trouble seeing with only one eye, or may be unable to tolerate an imbalance between their eyes. In addition to monovision, other approaches include bilateral correction with either bi-focal or multi-focal lenses. In the case of bi-focal lenses, the lens is made so that both a distant point and a near point can be focused. In the multi-focal case, there exist many focal points between near targets and far targets.

Surgical treatments have also been proposed for presbyopia. Anterior sclerostomy involves a surgical incision into the sclera that enlarges the ciliary space and facilitates movement of the lens. Also, scleral expansion bands (SEBs) have been suggested for increasing the ciliary space. Problems remain with such techniques, however, such as inconsistent and unpredictable outcomes.

In the field of refractive surgery, certain ablation profiles have been suggested to treat the condition, often with the goal of increasing the range of focus of the eye, as opposed to restoring accommodation in the patient's eye. Many of these ablation profiles can provide a single excellent focus of the eye, yet they do not provide an increased depth of focus such that optimal distance acuity, optimal near acuity, and acceptable intermediate acuity occur simultaneously. Shapes have been proposed for providing enhanced distance and near vision, yet current approaches do not provide ideal results for all patients. For example, one profile may optimize both distance and near vision when pupils are constricted, while providing only suboptimal acuity when pupils are dilated.

In light of the above, it would be desirable to have improved methods and systems for treatment and/or mitigation of presbyopia and other optical defects. Ideally, such methods and systems would provide for improved acuity without relying solely on one eye for distance vision and one eye for near vision. At least some of these objectives will be met by various embodiments of the present invention.

US 2004/0054356 describes methods and systems for laser treatment of presbyopia using offset imaging. As described, an offset image of a variable aperture such as a variable width slit and variable diameter iris diaphragm is scanned in a preselected pattern to perform ablative sculpting of predetermined portions of a corneal surface. The scanning is performed to ablate an optical zone sized to match the patient pupil with a peripheral transition zone outside the pupil. The optical zone is designed to correct for near-vision centrally and far-vision peripherally.

International Patent Application Number WO99/44492 describes an ophthalmic surgery system, and method for treating presbyopia by performing ablative photodecomposition of the corneal surface (200). The offset image of a variable aperture, such as a variable width slit, and variable diameter iris diaphragm, is scanned in a preselected pattern to perform ablative sculpting of predetermined portions of a corneal surface.

Aspects and examples of the invention are set out in the claims. Systems and methods of the present invention provide for treatment or amelioration of presbyopia. In one aspect of the invention, a method for treating presbyopia in a patient involves ablating a central zone of a corneal surface of a first eye of the patient to improve the patient's ability to view near objects through the central zone of the first eye and ablating a peripheral zone of a corneal surface of a second eye of the patient to improve the patients ability to view near objects through the peripheral zone of the second eye.

In some embodiments, the central zone produced during the first ablating step comprises a substantially spherical surface. Alternatively, the central zone may comprises a multifocal aspheric surface. Optionally, ablating the central zone of the corneal surface of the first eye may involve leaving a small central portion of the corneal surface untreated. In some embodiments, the ablated central zone may have a diameter scaled to a diameter of a pupil of the first eye. The ablated central zone may have any desired optical power, but in some embodiments it has an optical power of between about 0.5 and 4.0 Diopters (D), and more preferably between about 1.0 and 3.0 D, and even more preferably an optical power of about 1.75 D.

A method described herein further includes ablating a peripheral zone of a corneal surface of the first eye to improve the patient's ability to view far objects through the peripheral zone of the first eye. For example, in some embodiments the peripheral zone of the first eye extends radially outward from an outer boundary of the ablated central zone of the first eye to a diameter approximately matching an outer boundary of a pupil of the first eye. The method may optionally further include ablating a transition zone of the corneal surface of the first eye, the transition zone extending from an outer boundary of the ablated peripheral zone of the first eye.

Optionally, ablating the peripheral zone of a corneal surface of the second eye may involve leaving a central zone of the corneal surface of the second eye untreated to provide for vision of distant objects through the central zone. The method may include ablating a central zone of the corneal surface of the second eye to improve the patient's ability to view distant objects through the central zone.

Also described herein is a method for performing laser eye surgery on a patient to treat presbyopia involves: determining a first ablative shape for a corneal surface, the first ablative shape enhancing vision of near objects through a central zone of an eye; ablating a corneal surface of a first eye of the patient according to the first ablative shape; determining a second ablative shape for a corneal surface, the second ablative shape enhancing vision of near objects through a peripheral zone of an eye; and ablating a corneal surface of a second eye of the patient according to the second ablative shape.

In some embodiments, the first ablative shape comprises a central zone having a substantially spherical shape, while in other embodiments the first ablative shape comprises a central zone having a multifocal aspheric surface. Optionally, the first ablative shape may include a small central portion of the central zone that remains untreated. In some embodiments, the central zone of the first ablative shape has a diameter scaled to a diameter of a pupil of the first eye.

In some embodiments, the central zone of the eye according to the first ablative shape has an optical power of between about 0.5 and 4.0 D, more preferably between about 1.0 and 3.0 D, and even more preferably about 1.75 D. In some embodiments, the first ablative shape includes a peripheral zone shaped to provide for vision of distant objects. For example, the peripheral zone in some embodiments extends radially outward from an outer boundary of the central zone of the first ablative shape. Optionally, the first ablative shape may further include a transition zone extending from an outer boundary of the peripheral zone.

In some embodiments, the peripheral zone of the second ablative shape extends circumferentially around a center of the corneal surface. In some embodiments, the second ablative shape includes an untreated central zone to provide for vision of distant objects. In other embodiments, the second ablative shape includes a central zone shaped to improve the patient's ability to view distant objects.

A laser eye surgery system is provided for treating presbyopia in a patient includes a laser device for emitting a beam of ablative energy and a processor coupled with the laser device to direct the beam of ablative energy to ablate a first ablative shape on a corneal surface of a first eye of the patient and a second ablative shape on a corneal surface of a second eye of the patient. The first ablative shape enhances near vision through a central zone of the first eye, and the second ablative shape enhances near vision through a peripheral zone of the second eye. These first and second ablative shapes may have any of the features of the first and second ablative shapes described above.

These and other aspects and embodiments of the present invention are described in further detail below, in reference to the attached drawing figures.

### BRIEF DESCRIPTION OF THE DRAWINGS

FIG. 1 is a diagrammatic illustration of two different ablation shapes, each shape for a different eye of the same patient, according to one embodiment of the present invention.
FIGS. 2A and 2B are diagrammatic illustrations of two different power profiles resulting from ablation shapes such as those shown in FIG. 1, according to one embodiment of the present invention.
FIG. 3 is a side sectional view of an eye treated to enhance vision of near objects through a central zone of the eye, according to one embodiment of the present invention.
FIG. 4 illustrates an ablation profile on a corneal surface for enhancing vision of near objects through a central zone of the eye, according to one embodiment of the present invention.
FIG. 5 is a side sectional view of an eye treated to enhance vision of near objects through a peripheral zone of the eye, according to one embodiment of the present invention.
FIG. 6 illustrates an ablation profile on a corneal surface for enhancing vision of near objects through a peripheral zone of the eye, according to one embodiment of the present invention.
FIG. 7 is a block diagram of an ophthalmic surgery system for incorporating the invention.

### DETAILED DESCRIPTION OF THE INVENTION

While systems of the present invention are described primarily in the context of improving laser eye surgery methods and systems, various embodiments may also be adapted for use in alternative eye treatment procedures and systems such as femtosecond lasers and laser treatment, infrared lasers and laser treatments, radial keratotomy (RK), scleral bands, follow up diagnostic procedures, and the like. In other embodiments, techniques and systems of the present invention may be adapted for use in other eye treatment procedures and systems, such as contact lenses, intra-ocular lenses, radial keratotomy, collagenous corneal tissue thermal remodeling, removable corneal lens structures, glass spectacles and the like.

The present invention is particularly useful for enhancing laser eye surgical procedures such as photorefractive keratectomy (PRK), phototherapeutic keratectomy (PTK), laser in situ keratomileusis (LASIK), and the like. Various embodiments provide enhance presbyopia correction approaches by using improved combinations of ablation shapes for a patient's eyes.

The techniques described herein can be readily adapted for use with existing laser systems, including the VISX Excimer laser eye surgery systems commercially available from VISX of Santa Clara, California. By utilizing two different corneal ablation profiles for two different eyes of a patient, the present invention may enhance treatment of presbyopia.

A first eye of a patient is ablated to have a shape that enhances vision of near objects through a central region (or "central zone") of the first eye. A number of different ablation shapes and techniques may be used in various embodiments, such as shapes/techniques described in U.S. Patent No. 6,280,435, U.S. Patent No. 6,663,619 and/or U.S. Patent Application Serial No. 10/738,358 (Attorney Docket No. 018158-022220US), all of which are assigned to the assignee of the present invention, and all of which are hereby fully incorporated by reference.

The second eye of the patient is ablated to have a shape that enhances vision of near objects through a peripheral region (or "peripheral zone") of the second eye. Any suitable ablation techniques or shapes may be used, according to various embodiments. In one embodiment, for example, an ablation technique and shape as described in U.S. Patent Application Serial No. 09/841,674 (Publication No. 2002/0156467) may be used.

By ablating two eyes of a patient to achieve different ablation shapes, techniques provide for enhanced treatment of presbyopia. The patient will typically view both near and distant objects with both eyes. As the patient's pupils constrict, one eye will predominate for near vision and the other will predominate for distance vision. As the patient's pupils dilate, the predominant near and distance vision eyes will switch. The combination of the two ablation shapes enhances the patient's ability to view near, far and intermediate objects with an acceptable amount of acuity and without requiring bifocals or monovision systems.

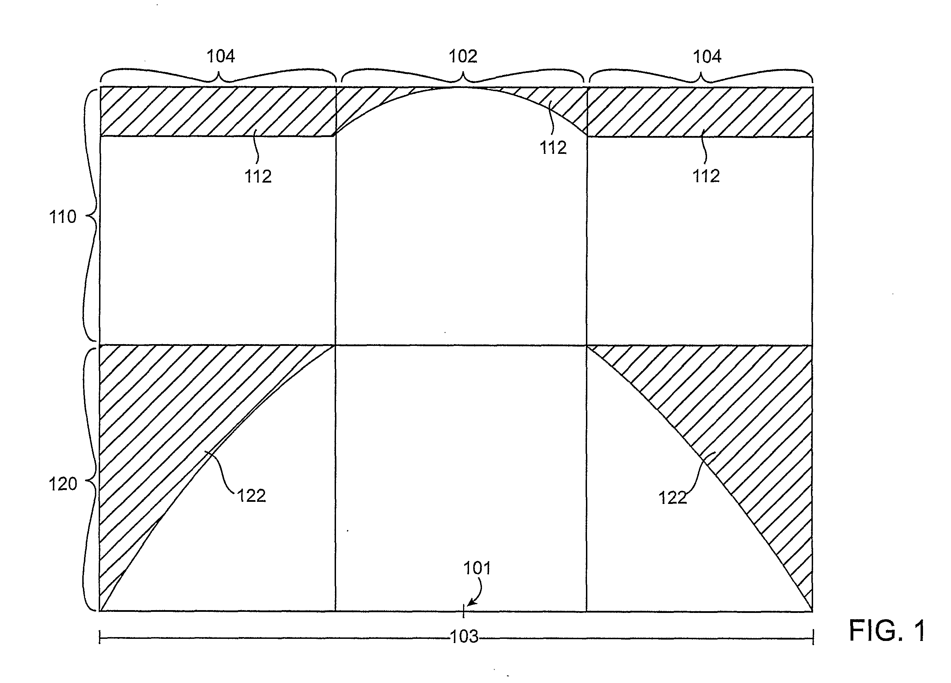

Turning now to the drawings, FIG. 1 illustrates a first ablation profile 110, which may be applied to a first eye of a patient, and a second ablation profile 120, which may be applied to a second eye of the same patient. In some patients, the first profile 110 may be used for the patient's left eye and the second profile 120 may be used for the right eye, while in other patients the profiles maybe used for the opposite eyes. Furthermore, the profiles shown in FIG. 1 are diagrams used solely for illustrative purposes. They are not drawn to scale and do not limit actual ablation profiles used in various embodiments of the invention in anyway.

That being said, FIG. 1 illustrates ablative shapes 110, 120 along a pupil 103 of each of two eyes of a patient, each pupil 103 having a pupil center 101. In both diagrams of the ablative shapes 110, 120, the hash-marked areas represent tissue removed 112, 122 from a corneal surface by ablation, typically by laser. Both ablative shapes 110, 120, include a central zone 102 and a peripheral zone 104. In the first ablative shape 110, used for a first eye of a patient, the removed tissue 112 creates a shape that enhances near vision through the central zone 102 and distance vision through the peripheral zone 104. In the second ablative shape 120, used for a second eye of the patient, the removed tissue 122 creates a shape that enhances distance vision through the central zone 102 and near vision through the peripheral zone 104. These ablation shapes 110, 120 may be used on the left and right eyes of the same patient, so that near and distance vision is enhanced through different portions of each eye.

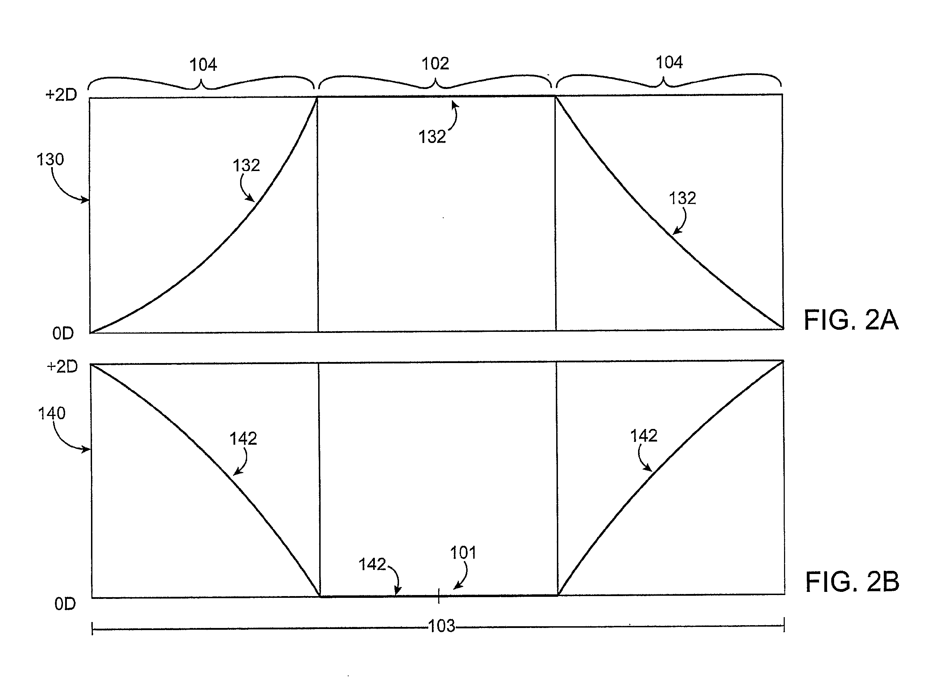

Referring now to FIGS. 2A and 2B, two power diagrams 130,140 illustrate dioptic powers of the two ablation shapes in FIG. 1, with a first power diagram 130 of FIG. 2A corresponding to the first ablation shape 110, and a second power diagram 140 of FIG. 2B corresponding to the second ablation shape 120. In FIG. 2A, the first power diagram 130 shows that power 132 increases toward +2 diopters (+2D) from the outer edge of the peripheral zone 104 toward the central zone 102 with the first ablative shape 110. In FIG. 2B, the second power diagram shows that power 132 decreases from +2 diopters (+2D) from the outer edge of the peripheral zone 104 toward the central zone 102 with the second ablative shape 120.

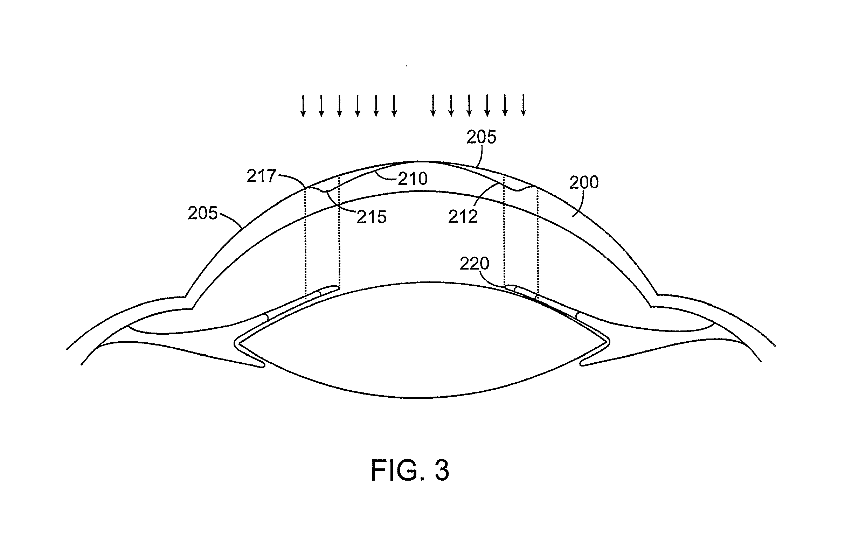

Referring now to FIG. 3, a schematic side view of a cornea 200 treated according to one embodiment is illustrated. The cornea 200 has an anterior surface that provides most of the refractive power of the eye. The initial anterior surface 205 of the cornea 200 has been reshaped to a desired profile. The desired profile includes anterior optical surface 210 and anterior transition surface 215. The anterior optical surface 210 has a multifocal aspheric shape that corrects for near vision centrally and far vision peripherally. Such a profile is similar to the first ablation profile 110 in FIG. 1.

While the present invention will often be described with reference to the mitigation of presbyopia in combination with refractive hyperopia treatment, the benefits of the present invention are not limited to these specific procedures. These presbyopia treatment techniques may be used when no other refractive correction (other than the correction, mitigation, and/or inhibition of presbyopia) is desired, or the present treatment may be combined with therapies for one or more of myopia, astigmatism, irregular refractive aberrations, and the like, as well as with hyperopia. Still other aspects of the present invention, including methods and systems which accommodate and adjust for re-epithelization, may find uses in a broad variety of ophthalmic procedures.

Anterior transition surface 215 is the anterior surface of the cornea that provides a gradual change in shape between anterior optical surface 210 and the portion of the cornea retaining the initial anterior surface 205. The outer boundary 212 of the anterior optical surface preferably extends entirely across, and is ideally substantially coextensive with, the pupil which is bounded by iris 220. The light rays passing through anterior transition surface 215 do not contribute to the image formed by anterior optical surface 210. Therefore, anterior transition surface 215 is desirably positioned outside the pupil. This positioning of anterior transition surface 215 causes the light rays passing through anterior transition surface 215 to be substantially occluded by iris 220. This occlusion improves patient vision because the light rays are blocked that do not contribute to image formation, and which would otherwise reduce the contrast of the image.

The optical correction effected by an ablative surgical procedure to the cornea is derived from a change in the anterior corneal surface from an initial anterior surface 205 to post-operative anterior optical surface 210. The anterior optical correction is the post-operative anterior optical surface 210 minus the initial anterior surface 205. An ablation profile is a change in an exposed surface profile occurring immediately after the tissue removal process. Therefore, the ablation profile is the exposed surface profile immediately after the tissue removal process minus the initial exposed surface profile. As used herein, "ablated shape" or "ablative shape" can refer either to an ablation-induced change in a surface topography on a surface of the cornea, or to the surface topography of the cornea after ablation.

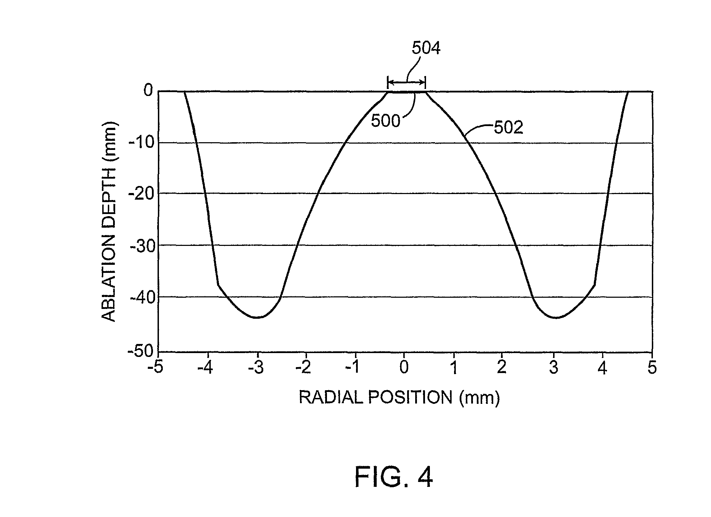

In some instances, it may be desirable to form a central add while leaving a central region of the optical zone untreated as illustrated in FIG. 4. A small untreated zone 500 centered on the optical zone 502 of an ablated cornea has a dimension 504 across the untreated zone. The untreated zone 504 is smoothed by covering and healing of the cornea and contributes to the formation of a central anterior optical surface that corrects presbyopia.

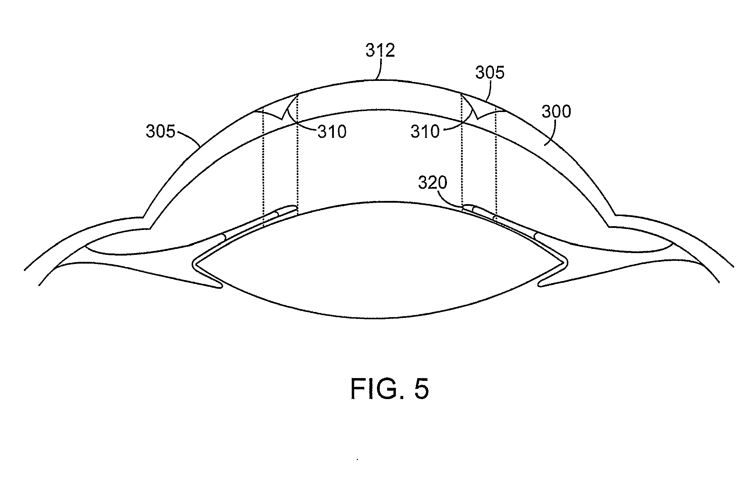

Referring now to FIG. 5, a schematic side view of a cornea 300 treated to achieve peripheral add, according to one embodiment, is shown. The cornea 300 has an anterior surface that provides most of the refractive power of the eye. The initial anterior surface 305 of the cornea 300 has been reshaped to a desired profile. The desired profile includes anterior optical surface 305 that corrects for near-vision peripherally and far-vision centrally. To achieve the desired profile, anterior optical surface 305 is ablated lateral to pupil, which is bounded by iris 320. In some embodiments, a central zone 312 of the corneal surface 305 is not ablated, thus providing for distance vision through central zone 312. In other embodiments, central zone 312 may be ablated to enhance distance vision through central zone 312. The profile shown here is similar to the second ablative profile 120 illustrated in FIG. 1.

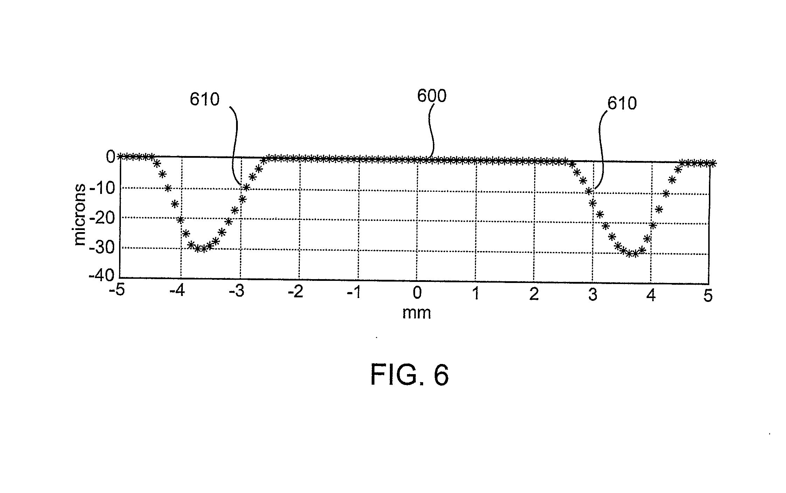

FIG. 6 schematically shows an ablation shape for providing peripheral add as just described. As can be seen from the figure, a central zone 600, having a radius of about 5.0 mm, is untreated, while a peripheral zone 610 is ablated to enhance near vision. The untreated central zone 600 is then used primarily for distance vision.

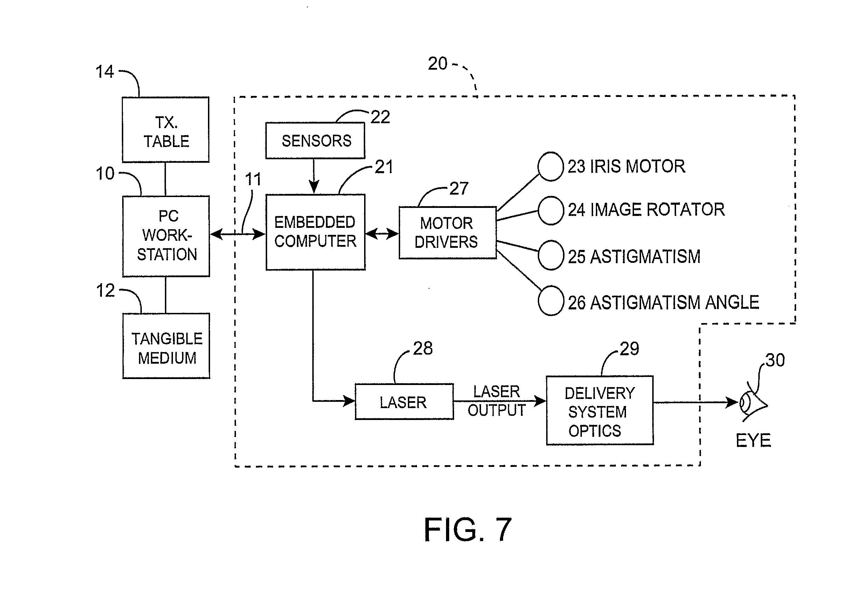

FIG. 7 illustrates a block diagram of an ophthalmic surgery system for incorporating the invention. As seen in this Figure, a personal computer (PC) work station 10 is coupled to an embedded computer 21 of a laser surgery unit 20 by means of a first bus connection 11. The PC work station 10 comprises a tangible medium 12 and a treatment table 14. Tangible medium 12 will typically comprise a memory, magnetic recording media, an optical disk, or the like, and will generally comprise machine readable programming instruction code implementing the method steps described herein. The laser treatment table 14 includes a listing of coordinate references of the laser beam during an ablation of the cornea. The subcomponents of laser surgery unit 20 are known components and preferably comprise the elements of the VISX START™ Excimer Laser Systems, such as the STAR S4™ System, available from VISX, Incorporated of Santa Clara, California. Thus, the laser surgery system 20 includes a plurality of sensors generally designated with reference numeral 22 which produce feedback signals from the movable mechanical and optical components in the laser optical system, such as the elements driven by an iris motor 23, an image rotator 24, an astigmatism motor 25 and an astigmatism angle motor 26. The feedback signals from sensors 22 arc provided via appropriate signal conductors to the embedded computer 21. The embedded computer 21 controls the operation of the motor drivers generally designated with reference numeral 27 for operating the elements 23-26. In addition, embedded computer 21 controls the operation of the excimer laser 28, which is preferably an argon-fluorine laser with a 193 nanometer wavelength output designed to provide feedback stabilized fluence of 160 mJoules per square centimeter at the cornea of the patient's eye 30 via the delivery system optics generally designated with reference numeral 29. In addition, other suitable laser systems may be utilized in the present invention including, for example, those manufactured by Alcon, Bausch & Lomb, Wavelight, Nidek, Schwind, Zeiss-Meditec, Lasersight, and the like. Other lasers having a suitable wavelength may be used to make an ablative energy for removing a tissue from the eye. For example, solid state lasers such as a yttrium aluminum garnet (YAG) laser producing a fifth harmonic of a fundamental wavelength may be used to generate an ablative energy. Other ancillary components of the laser surgery system 20 which are not necessary to an understanding of the invention, such as a high resolution microscope, a video monitor for the microscope, a patient eye retention system, and an ablation effluent evacuator/filter, as well as the gas delivery system, have been omitted to avoid prolixity. Similarly, the keyboard, display, and conventional PC subsystem components (e.g., flexible and hard disk drives, memory boards and the like) have been omitted from the depiction of the PC work station 10. If desired, embedded computer 21 may be constructed with PC work station components and built into laser surgery system 20. In this case embedded computer 21 may supplant PC workstation 10.

While the above provides a full and complete disclosure of the preferred embodiments of the invention, various modifications, alternate constructions and equivalents may be employed as desired. Therefore, the above description and illustrations should not be construed as limiting the invention, which is defined by the appended claims.

## Claims

1. A laser eye surgery system for treating presbyopia in a patient, the system comprising:
a laser device for emitting a beam of ablative energy; and
a processor coupled with the laser to direct the beam of ablative energy to ablate a first ablative shape on a corneal surface of a first eye of the patient and a second ablative shape on a corneal surface of a second eye of the patient, wherein the processor includes a tangible medium having a treatment table associated with each ablative shape embodied thereon, and wherein the first ablative shape enhances near vision through a central zone of the first eye, and the second ablative shape enhances near vision through a peripheral zone of the second eye and wherein the first ablative shape further comprises a peripheral zone for viewing distant objects and the second ablative shape includes central zone to provide for vision of distant objects.

2. A system as in claim 1, wherein the treatment table includes reference coordinates for directing the laser device to ablate the first and second ablative shapes.

3. A system as in claim 1, wherein the treatment table is configured so that the central zone of the first ablative shape comprises a substantially spherical surface.

4. A system as in claim 1, wherein the treatment table is configured so that the central zone of the first ablative shape comprises a multifocal aspheric surface.

5. A system as in claim 1, wherein the treatment table is configured so that the first ablative shape includes a small untreated central portion within the central zone.

6. A system as in claim 1, wherein the treatment table is configured so that the central zone of the first ablative shape has a diameter scaled to a diameter of a pupil of the first eye.

7. A system as in claim 1, wherein the treatment table is configured so that the central zone of the first ablative shape has an optical power of between about 0.5 and 4.0 Diopters.

8. A system as in claim 7, wherein the treatment table is configured so that the central zone has an optical power of between about 1.0 and 3.0 Diopters.

9. A system as in claim 8, wherein the treatment table is configured so that the central zone has an optical power of about 1.75 Diopters.

10. A system as in claim 8, wherein the treatment table is configured so that the first ablative shape further includes a transition zone, the transition zone extending from an outer boundary of the peripheral zone.

## Patentansprüche

1. Laseraugenchirurgie-System zum Behandeln von Presbyopie in einem Patienten, wobei das System umfasst:
eine Laservorrichtung zum Emittieren eines Strahls von Ablationsenergie; und
einen Prozessor, der mit dem Laser gekoppelt ist, um den Strahl von Ablationsenergie zu führen zum Ablatieren einer ersten Ablationsform auf einer Hornhautfläche eines ersten Auges des Patienten und einer zweiten Ablationsform auf einer Hornhautfläche eines zweiten Auges des Patienten, wobei der Prozessor ein reales Medium mit einer Behandlungstabelle aufweist, der jeder darin enthaltenen Ablationsform zugeordnet ist, und wobei die erste Ablationsform das Nahsehen durch eine zentrale Zone des ersten Auges verbessert und die zweite Ablationsform das Nahsehen durch eine periphere Zone des zweiten Auges verbessert und wobei die erste Ablationsform ferner eine periphere Zone zum Sehen von entfernten Objekten umfasst und die zweite Ablationsform eine zentrale Zone zum Ermöglichen des Sehens von entfernten Objekten aufweist.

2. System nach Anspruch 1, wobei die Behandlungstabelle Referenzkoordinaten zum Führen der Laservorrichtung zum Ablatieren der ersten und der zweiten Ablationsform aufweist.

3. System nach Anspruch 1, wobei die Behandlungstabelle so ausgelegt ist, dass die zentrale Zone der ersten Ablationsform eine im Wesentlichen sphärische Fläche umfasst.

4. System nach Anspruch 1, wobei die Behandlungstabelle so ausgelegt ist, dass die zentrale Zone der ersten Ablationsform eine multifokale asphärische Fläche umfasst.

5. System nach Anspruch 1, wobei die Behandlungstabelle so ausgelegt ist, dass die erste Ablationsform einen kleinen unbehandelten zentralen Abschnitt innerhalb der zentralen Zone aufweist.

6. System nach Anspruch 1, wobei die Behandlungstabelle so ausgelegt ist, dass die zentrale Zone der ersten Ablationsform einen Durchmesser aufweist, der auf einen Durchmesser einer Pupille des ersten Auges skaliert ist.

7. System nach Anspruch 1, wobei die Behandlungstabelle so ausgelegt ist, dass die zentrale Zone der ersten Ablationsform eine optische Stärke zwischen ungefähr 0,5 und 4,0 Dioptrien aufweist.

8. System nach Anspruch 7, wobei die Behandlungstabelle so ausgelegt ist, dass die zentrale Zone eine optische Stärke zwischen ungefähr 1,0 und 3,0 Dioptrien aufweist.

9. System nach Anspruch 8, wobei die Behandlungstabelle so ausgelegt ist, dass die zentrale Zone eine optische Stärke von ungefähr 1,75 Dioptrien aufweist.

10. System nach Anspruch 8, wobei die Behandlungstabelle so ausgelegt ist, dass die erste Ablationsform ferner eine Übergangszone aufweist, wobei sich die Übergangszone von einer Außengrenze der peripheren Zone erstreckt.

## Revendications

1. Système de chirurgie laser des yeux pour le traitement de la presbytie chez un patient, le système comprenant :
un dispositif laser destiné à émettre un faisceau d'énergie ablative ; et
un processeur couplé au laser destiné à diriger le faisceau d'énergie ablative pour retirer une première forme ablative sur une surface cornéenne d'un premier oeil du patient et une deuxième forme ablative sur une surface cornéenne d'un deuxième oeil du patient, le processeur comportant un support matériel ayant une table de traitement associée à chaque forme ablative représentée sur celle-ci, la première forme ablative améliorant la vision de près à travers une zone centrale du premier oeil et la deuxième forme ablative améliorant la vision de près à travers une zone périphérique du deuxième oeil, et la première forme ablative comprenant en outre une zone périphérique pour voir les objets distants et la deuxième forme ablative comportant une zone centrale pour fournir une vision des objets distants.

2. Système selon la revendication 1, dans lequel la table de traitement comporte des coordonnées de référence destinées à diriger le dispositif laser pour retirer les première et deuxième formes ablatives.

3. Système selon la revendication 1, dans lequel la table de traitement est configurée de telle sorte que la zone centrale de la première forme ablative comprend une surface sensiblement sphérique.

4. Système selon la revendication 1, dans lequel la table de traitement est configurée de telle sorte que la zone centrale de la première forme ablative comprend une surface asphérique multifocale.

5. Système selon la revendication 1, dans lequel la table de traitement est configurée de telle sorte que la première forme ablative comporte une petite portion centrale non traitée à l'intérieur de la zone centrale.

6. Système selon la revendication 1, dans lequel la table de traitement est configurée de telle sorte que la zone centrale de la première forme ablative a un diamètre proportionné au diamètre de la pupille du premier oeil.

7. Système selon la revendication 1, dans lequel la table de traitement est configurée de telle sorte que la zone centrale de la première forme ablative a une puissance optique comprise entre environ 0,5 et 4,0 dioptries.

8. Système selon la revendication 7, dans lequel la table de traitement est configurée de telle sorte que la zone centrale a une puissance optique comprise entre environ 1,0 et 3,0 dioptries.

9. Système selon la revendication 8, dans lequel la table de traitement est configurée de telle sorte que la zone centrale a une puissance optique d'environ 1,75 dioptrie.

10. Système selon la revendication 8, dans lequel la table de traitement est configurée de telle sorte que la première forme ablative comporte en outre une zone de transition, la zone de transition s'étendant depuis une limite extérieure de la zone périphérique.
